# EUROPÄISCHE PATENTSCHRIFT

(11) **EP 3 145 586 B1**
(45) Veröffentlichungstag und Bekanntmachung des Hinweises auf die Patenterteilung: **04.07.2018**
(21) Anmeldenummer: 15726073.8
(22) Anmeldetag: 22.05.2015
(51) Int. Cl.: A61N 5/06, A61B 18/00

(54) **OPTISCHE STIMULATIONSEINRICHTUNG UND VERFAHREN ZUR PROGRAMMIERUNG**
OPTICAL STIMULATION DEVICE AND METHOD FOR PROGRAMMING
DISPOSITIF DE STIMULATION OPTIQUE ET PROCÉDÉ DE PROGRAMMATION

(30) Priorität: 23.05.2014 DE 102014107298
(43) Veröffentlichungstag der Anmeldung: 29.03.2017
(73) Patentinhaber: Leibniz-Institut für Neurobiologie Magdeburg, 39118 Magdeburg (DE); Otto-von-Guericke-Universität Magdeburg, 39106 Magdeburg (DE); Otto-von-Guericke Universität Magdeburg Medizinische Fakultät, 39120 Magdeburg (DE)
(72) Erfinder: LIPPERT, Michael, 04277 Leipzig (DE); OHL, Frank, 39171 Osterweddingen (DE); JANITZKI, Kathrin, 39112 Magdeburg (DE); HEINZE, Hans-Joachim, 39120 Magdeburg (DE)
(74) Vertreter: Gramm, Lins & Partner Patent- und Rechtsanwälte PartGmbB
(86) Internationale Anmeldenummer: PCT/EP2015/061400
(87) Internationale Veröffentlichungsnummer: WO 2015/177345

(56) Entgegenhaltungen:
- US-A1- 2011 295 331
- US-A1- 2012 253 261
- US-A1- 2013 030 353
- US-A1- 2013 046 148
- JIAYI ZHANG ET AL: "Integrated device for optical stimulation and spatiotemporal electrical recording of neural activity in light-sensitized brain tissue", JOURNAL OF NEURAL ENGINEERING, INSTITUTE OF PHYSICS PUBLISHING, BRISTOL, GB, Bd. 6, Nr. 5, 1. September 2009 (2009-09-01), Seite 55007, XP020165975, ISSN: 1741-2552, DOI: 10.1088/1741-2560/6/5/055007

## Beschreibung

Die Erfindung betrifft eine optische Stimulationseinrichtung zur Stimulation von Nervenzellen gemäß dem Anspruch 1. Die Erfindung betrifft außerdem ein Verfahren zur Programmierung des Parameterspeichers einer Stimulationseinrichtung gemäß dem Anspruch 11.

Allgemein betrifft die Erfindung das Gebiet der optischen Stimulation von Nervenzellen, d.h. der Stimulation durch Licht. Die Erfindung betrifft damit das Gebiet der Optogenetik. Die Entwicklung der Optogenetik, der Sensibilisierung normaler Nervenzellen für Licht durch genetische Methoden, nähert die Hoffnung, eine Reihe von Erkrankungen durch zeitlich und räumlich selektive Aktivierung oder Inhibition von neuronalen Strukturen mit Licht behandeln zu können. Bisherige Systeme beruhen auf der stereotaktischen Platzierung von Elektroden und Lichtquellen direkt im Hirngewebe bzw. auf der Oberfläche des Gehirns. Dies geht mit aufwändigen chirurgischen Eingriffen und entsprechenden Risiken einher. Die notwendige stereotaktische Planung kompliziert darüber hinaus die breite Anwendbarkeit.

Außerdem führt die Implantation der lichterzeugenden Strukturen zu einem nicht zu vernachlässigenden Wärmeeintrag im Gehirn. Um eine Gewebeschädigung zu vermeiden, ist die mögliche erreichbare Leistungsdichte der Lichtsignale stark eingeschränkt.

Aus den Veröffentlichungen US 2012/0253261 A1, JIAYI ZHANG ET AL: "Integrated device for optical stimulation and spatiotemporal electrical recording of neural activity in light-sensitized brain tissue", Journal of neural engineering, Institute of physics publishing, Bristol, GB, Bd. 6, Nr. 5, 1, September 2009, Seite 55007, US 2013/046148 A1 und US 2013/030353 A1 gehen jeweils optische Stimulationseinrichtungen zur Stimulation von Nervenzellen hervor. Die US 2011/0295331 A1 betrifft ein Cochlea-Implantat.

Der Erfindung liegt daher die Aufgabe zugrunde, eine optische Stimulationseinrichtung zur Stimulation von Nervenzellen anzugeben, die bei vereinfachter Anwendbarkeit zugleich eine höhere Effizienz hat und dadurch den Weg zu einer breiteren Anwendbarkeit optogenetischer Behandlungsmethoden eröffnet.

Diese Aufgabe wird durch eine optische Stimulationseinrichtung gemäß Anspruch 1 gelöst.

Die Erfindung hat den Vorteil, dass erstmalig eine optische Stimulationseinrichtung zur Stimulation von Nervenzellen, insbesondere Nervenzellen des Gehirns, angegeben werden kann, die durch nichtinvasive oder minimalinvasive Operationsmethoden zum Einsatz gebracht werden kann. Insbesondere kann die Implantat-Komponente endoskopisch in dem gewünschten inneren Hohlraum des Körpers platziert werden und dort verankert werden, indem eine Fixierung in dem natürlichen inneren Hohlraum durch Expansion der Trägerstruktur in radialer Richtung erfolgt. Die Fixierung kann z.B. durch Ballonexpansion erfolgen, wie von Stents her bekannt. Zur Anbringung und Platzierung der Implantat-Komponente in dem inneren Hohlraum kann z.B. ein flexibler Führungsstab verwendet werden, z.B. in Form eines Katheters. Vorteilhaft kann z.B. ein Ventrikelraum zur Platzierung der Implantat-Komponente genutzt werden. In diesem Fall ist die Einbringung der Implantat-Komponente in den Ventrikelraum nichtinvasiv durchführbar, indem die Wand des Ventrikels nicht durchbrochen
wird, sondern die Platzierung der Implantat-Komponente über die Öffnungen des Ventrikelsystems erfolgt. Es ist auch eine minimalinvasive Platzierung möglich, wenn ein Durchstechen einer Hirnstruktur erfolgt, welche zu möglichst wenigen Nebenwirkungen führt. Die minimalinvasive oder nichtinvasive Natur der erfindungsgemäßen Stimulationseinrichtung erlaubt zudem ihren Austausch bei einem Defekt oder einer Weiterentwicklung der Stimulationseinrichtung. Auch eine Repositionierung ist möglich, ohne dass eine zusätzliche Schädigung im Hirngewebe auftritt.

Die offene Struktur der Trägerstruktur, d.h. die Öffnungen und/oder Kanäle, erlauben ein Durchströmen der Trägerstruktur und damit der Implantat-Komponente nach Implantation im Körper mittels der Körperflüssigkeit, so dass auf natürliche Weise eine Kühlung der Lichtquellen erfolgen kann. Auf diese Weise kann das eingangs genannte Wärmeproblem durch die Lichtquellen gelöst werden. Bei Platzierung der Implantat-Komponente im Ventrikelraum, wie erwähnt, kann direkt die Cerebrospinalflüssigkeit zur Kühlung der Lichtquellen genutzt werden. Hierdurch ist eine besonders effiziente Kühlung möglich, was erheblich höhere abstrahlbare Lichtleistungen ermöglicht. Zugleich können Schädigungen des Hirngewebes durch unzulässige Erwärmung vermieden werden.

Durch die Lichtabstrahlung in radialer Richtung bezüglich der Trägerstruktur wird das Licht direkt in das umgebende Gewebe abgegeben. Die Lichtabstrahlung muss dabei nicht ausschließlich exakt in radialer Richtung erfolgen. Um einen hohen optischen Wirkungsrad zu erzielen, ist es vorteilhaft, wenn die radiale Komponente der Lichtabstrahlrichtung die überwiegende Komponente ist.

Mittels der erfindungsgemäßen optischen Stimulationseinrichtung ist eine Behandlung der folgenden Erkrankungen möglich:
- Stimulation des Locus coeruleus:
- Neurodegenerative Erkrankungen wie die Parkinson- und Alzheimererkrankung, welche durch eine frühe Degeneration des LC gekennzeichnet sind,
- schwere psychiatrische Erkrankungsbilder, wie Major Depression, Korsakow Syndrom, Angststörungen, PTSD, die durch veränderte Entladungen des LC gekennzeichnet sind,
- Störungen der Wachheit/Locked-In-Syndroms durch Stimulation des Locus coeruleus bzw. des ARAS,
- bei Narkolepsis oder nach unilateralen cerebralen Infarkten oder Blutungen im Hinstammbereich,
- Störungen autonomer Regelkreise durch Stimulation/Inhibition des Hypothalamus,
- Essstörungen, Überaktivierung der HPA-Achse im Rahmen vieler Erkrankungen, therapieresistente Hypertonie,
- Suchterkrankungen und Psychosen durch indirekte Stimulation frontaler Cortexareale via Aktivierung monoaminerger aszendierender Bahnen (z.B. des medialen Vorderhirnbündels, das von der VTA und dem LC zum Nucleus accumbens zieht),
- Neurodegenerative Erkrankungen/Gedächtnisstörungen durch Beeinflussung des Hippocampus (Platzierung der Implantat-Komponente im Unterhorn des Seitenventrikels)

Auch ohne genetische Beeinflussung kann die erfindungsgemäße optische Stimulationseinrichtung vorteilhaft eingesetzt werden. Dies wird möglich durch das Vorhandensein endogener lichtsensitiver Nervenzellen im Gehirn von Wirbeltieren. Zusätzlich können thermische Effekte von Infrarotlicht zur fokalen Erwärmung von bestimmten Hirnstrukturen genutzt werden, um deren Aktivität zu modulieren. Ebenfalls möglich ist eine Nutzung der Suppression neuronaler Aktivität durch die bleichenden Eigenschaften von blauem und grünem Licht auf die Flavoproteine der Atmungskette.

Durch eine direkte Lichtstimulation ohne hinzugefügte optogenetische Proteine erscheint z.B. eine Behandlung von therapieresistenten schweren Depressionen möglich.

Gemäß einer vorteilhaften Weiterbildung der Erfindung weist die Implantat-Komponente eine Steuerelektronik auf oder ist über Leitungen mit einer entfernt von der Implantat-Komponente angeordneten Steuerelektronik verbunden, wobei die Steuerelektronik zur Signalkonditionierung und/oder Signalverarbeitung der über die Elektroden aufgenommenen Signale und/oder zur Ansteuerung der Lichtquellen eingerichtet ist. Die Steuerelektronik kann einteilig oder mehrteilig ausgebildet sein, z.B. in Form einer direkt auf oder an der Implantat-Komponente angeordneten Headstage-Elektronik und einer entfernt angeordneten Hauptelektronik, die über Leitungen mit der Implantat-Komponente und insbesondere dessen Headstage-Elektronik verbunden ist. Auf diese Weise kann die erforderliche Elektronik soweit wie notwendig direkt an der Implantat-Komponente angeordnet werden, z.B. um eine unmittelbare Signalkonditionierung der Signale der Elektroden zu ermöglichen. Hierdurch kann die Headstage-Elektronik baulich klein gehalten werden. Weitere erforderliche Bauteile können dann in der Hauptelektronik untergebracht werden.

Die Lichtquellen können elektrische Lichtquellen sein, z.B. in Form von Halbleiterlichtquellen, insbesondere in Form von Leuchtdioden (LED) oder Laserdioden. Die Lichtquellen können auch passive Lichtquellen sein, z.B. in Form von Lichtaustrittsflächen von Lichtleitern oder an Lichtleitern angeordneten Prismen, Mikrospiegeln oder sonstige Optiken. Im Falle passiver Lichtquellen können die zu den Lichtquellen führenden Leitungen insbesondere Lichtleiter sein, z.B. in Form von Glasfasern. Dies hat den Vorteil, dass die optische Stimulationseinrichtung MRT-tauglich ist, d.h. die Aufnahmen mittels eines Magnetresonanztomographen (MRT) nicht stört. Sofern als Lichtquellen elektrische Lichtquellen, die an oder in der Implantat-Komponente angeordnet sind, eingesetzt werden, werden als Leitungen elektrische Leiter (Kabel) eingesetzt. Die an der Trägerstruktur befestigten Lichtquellen können auch eine Kombination aus elektrischen Lichtquellen und passiven Lichtquellen sein.

Gemäß einer vorteilhaften Weiterbildung der Erfindung sind die Lichtquellen in vordefinierter Lage bezüglich der Elektroden an der Trägerstruktur befestigt.

Gemäß einer vorteilhaften Weiterbildung der Erfindung weist die Steuerelektronik einen Parameterspeicher auf, in dem für jede Lichtquelle eine Information gespeichert ist, ob die Lichtquelle zu Stimulationszwecken aktivierbar ist oder nicht. Dies hat den Vorteil, dass die Platzierung der Implantat-Komponente in dem inneren Hohlraums des Körpers vereinfacht wird, da keine besonders hohe Positioniergenauigkeit erforderlich ist. Stattdessen kann die Implantat-Komponente mit den Lichtquellen sozusagen "überbestückt" sein, d.h. mehr Lichtquellen aufweisen als für die eigentliche Nervenstimulation erforderlich ist. Durch die bekannte Lage der Lichtquellen bezüglich der Elektroden an der Trägerstruktur kann durch probeweises Ansteuern einzelner Lichtquellen und Empfang der Elektrodensignale getestet werden, welche Lichtquelle am Günstigsten bezüglich der Nervenzellen ausgerichtet sind. Für diese kann dann in dem Parameterspeicher die Information gespeichert werden, dass die Lichtquelle zur Stimulationszwecken aktivierbar ist. Für die übrigen Lichtquellen kann die Information gespeichert werden, dass sie zu Stimulationszwecken nicht aktivierbar ist und dementsprechend im späteren Betrieb der optischen Stimulationseinrichtung ausgeschaltet bleibt. Hierdurch werden die elektrische Aktivierung und damit der hiermit verbundene Wärmeeintrag auf das notwendige Maß begrenzt. Es erfolgt keine unnötige Aktivierung von Lichtquellen, die keinen oder keinen wesentlichen Einfluss auf die Nervenzellen haben.

Die Identifikation der aktivierbaren Lichtquellen kann durch Messung über die Elektroden selbst oder durch eine anderweitig gemessene Antwort durchgeführt werden, z.B. durch EEG, Verhaltensantwort, funktionelle Kernspintomographie, PET, SPECT, pharmakologische Methoden. Wird die Identifikation der aktivierbaren Lichtquellen nicht durch Messung über die Elektroden durchgeführt, ist es nicht unbedingt erforderlich, die Lichtquellen in vordefinierter Lage bezüglich der Elektroden an der Trägerstruktur anzuordnen.

Gemäß einer vorteilhaften Weiterbildung der Erfindung weist die Trägerstruktur im nichtexpandierten Zustand einen Umfang von weniger als 10 mm auf, insbesondere weniger als 6 mm. Dies hat den Vorteil, dass eine minimalinvasive oder nichtinvasive Platzierung der Implantat-Komponente bei nahezu jedem Körper eines Lebewesens möglich ist.

Gemäß einer vorteilhaften Weiterbildung der Erfindung weist die Trägerstruktur ein Material mit hoher Wärmeleitfähigkeit auf. Insbesondere ist die Wärmeleitfähigkeit größer als 10 W/m•K. Dies hat den Vorteil, dass die Trägerstruktur zusätzlich zur Kühlung beiträgt, indem sie die an den Lichtquellen entstehende Wärme besser auf die als Kühlflüssigkeit dienende Körperflüssigkeit überträgt, die sich im inneren Hohlraum zusammen mit der Implantat-Komponente befindet.

Gemäß einer vorteilhaften Weiterbildung der Erfindung weisen eine, mehrere oder alle Lichtquellen thermisch leitfähige Kühlkörper auf. Hierdurch wird die Wärmeableitung von den Lichtquellen weiter verbessert.

Gemäß einer vorteilhaften Weiterbildung der Erfindung weist die Implantat-Komponente ein Kupplungselement auf, über das die Implantat-Komponente mit einem zur Durchführung des Implantationsvorgangs mit der Implantat-Komponente zu koppelnden flexiblen Führungsstab koppelbar ist, wobei die Kopplung des Kupplungselements mit dem flexiblen Führungsstab durch Betätigung eines Entriegelungsmechanismus lösbar ist. Dies ist zusätzlich förderlich für eine minimalinvasive oder nichtinvasive Platzierung der Implantat-Komponente im inneren Hohlraum des Körpers. Der flexible Führungsstab kann z.B. wie ein Endoskop oder ein Katheter ausgebildet sein. Durch den Entriegelungsmechanismus, der z.B. von einem Handgriff des flexiblen Führungsstabs fernbedienbar sein kann, kann nach korrekter Platzierung der Implantat-Komponente in dem inneren Hohlraum des Körpers der Führungsstab davon gelöst werden und wieder aus dem Körper entfernt werden. Zuvor kann über einen Expansionsmechanismus, der ebenfalls über den flexiblen Führungsstab auslösbar ist, die Trägerstruktur zur Fixierung der Implantat-Komponente in dem inneren Hohlraum des Körpers expandiert werden.

Gemäß einer vorteilhaften Weiterbildung der Erfindung sind einige oder alle Lichtquellen individuell aktivierbar. Dies hat den Vorteil, dass durch gezielte Ansteuerung bestimmter Lichtquellen besondere Behandlungsverfahren realisiert werden können, die bei simultaner Ansteuerung aller Lichtquellen nicht möglich wären. Hierdurch wird die Einsatzbreite der optischen Stimulationseinrichtung weiter verbessert.

Gemäß einer vorteilhaften Weiterbildung der Erfindung sind die Lichtquellen und/oder die Elektroden auf einem flexiblen Trägermaterial befestigt, das an der Trägerstruktur befestigt ist. Das flexible Trägermaterial kann z.B. ein Silikonschlauch oder eine MEMS-Struktur sein. Das flexible Trägermaterial ist dabei zumindest soweit mit Öffnungen und/oder Kanälen ausgebildet, dass der gewünschte Durchfluss der Körperflüssigkeit gewährleistet ist. Hierdurch wird die Herstellbarkeit der optischen Stimulationseinrichtung verbessert. So können die Lichtquellen, z.B. in Form von Leuchtdioden oder Laserdioden, auf eine Folie oder ein Silikonmaterial aufgedrückt werden und/oder fadenartig, ähnlich einer Schnur, auf die Trägerstruktur aufgefädelt werden.

Die Erfindung betrifft gemäß Anspruch 11 ferner ein Verfahren zur Programmierung des Parameterspeichers einer Stimulationseinrichtung der zuvor genannten Art, wobei zunächst alle Lichtquellen zur Lichtabgabe betätigt werden, die daraufhin mittels der Elektroden erfassten Signale ausgewertet werden oder durch andere Nervensignal-Erfassungsmethoden erfasste Signale ausgewertet werden, hieraus zukünftig aktivierbare Lichtquellen aus der Gesamtmenge der vorhandenen Lichtquellen bestimmt werden und für diese Lichtquellen im Parameterspeicher die Kennung "aktivierbar" gespeichert wird und für alle übrigen Lichtquellen die Kennung "nicht-aktivierbar" gespeichert wird. Mit diesem Verfahren vereinfacht sich die genaue Platzierung und Justierung der Implantat-Komponente in dem inneren Hohlraum des Körpers, da zunächst nur eine Grobplatzierung in Bezug auf die stimulierenden Nervenzellen erforderlich ist. Durch die beschriebene Programmierung des Parameterspeichers werden dann die Lichtquellen der Implantat-Komponente detektiert, die eine funktionelle Wirkung auf die Stimulation der Nervenzellen haben. Die übrigen Lichtquellen werden dann fortan nicht benötigt und können als "nicht-aktivierbar" gekennzeichnet werden. Eine weitere mechanische Feinplatzierung der Implantat-Komponente ist dann nicht erforderlich. Ebenfalls beschrieben wird ein Verfahren zum Implantieren einer optischen Stimulationseinrichtung in einem Körper eines Menschen oder Tieres, wobei eine Implantat-Komponente der optischen Stimulationseinrichtung endoskopisch in einem Ventrikelraum des Körpers platziert und dort fixiert wird. Hierbei kann insbesondere eine Implantat-Komponente einer optischen Stimulationseinrichtung der zuvor beschriebenen Art eingesetzt werden. Hierdurch können die zuvor erwähnten Vorteile realisiert werden.

Gemäß einer vorteilhaften Weiterbildung der Erfindung erfolgt das Fixieren der Implantat-Komponente im Ventrikelraum durch Expandieren einer Trägerstruktur der Implantat-Komponente.

Zur endoskopischen Platzierung des Katheters wird folgendes Verfahren, ausgeführt am Beispiel aquäduktnaher Zielstrukturen, vorgeschlagen: Es wird wie beim endoskopischen Stenting einer Aquäduktstenose zunächst eine kleine Trepanation (1,5 bis 2 cm im Durchmesser) ca. 2 bis 4 cm anterior der koronaren Sutur und 2 cm rechts der Mittellinie vorgenommen. Anschließend wird über ein rigides oder flexibles Neuroendoskop das Vorderhorn des rechten lateralen Ventrikels erreicht und schließlich ein French Fogarty-Ballon-Katheter über das Foramen Monroe und den III. Ventrikel in das Aquädukt eingebracht. Die exakte Platzierung der lichtemittierenden Spitze des Katheters, in Bezug auf die Zielstruktur, erfolgt entsprechend einer vorher tomographisch genau bestimmten Distanz zu einem Fixpunkt im Aquädukt. Fixiert wird der Katheter über die Expansion des Ballons im Aquädukt. So können Zielstrukturen in der Umgebung des IV. Ventrikels, insbesondere der Locus coeruleus erreicht werden. Die Navigation kann vorher mittels Software trainiert werden.

Ein anderer möglicher Weg zum IV. Ventrikel ist von occipital via der natürlichen Öffnungen zum äußeren Liquorraum. Durch die Apertura mediana ventriculi quarti ist der IV: Ventrikel mit der Cisterna cerebollomedullaris verbunden und durch die Apertura lateralis beidseits mit der Cisterna pontis. Positionierung und Verankerung erfolgen analog der vorher beschriebenen Weise.

Die Erfindung wird nachfolgend anhand von Ausführungsbeispielen unter Verwendung von Zeichnungen näher erläutert.

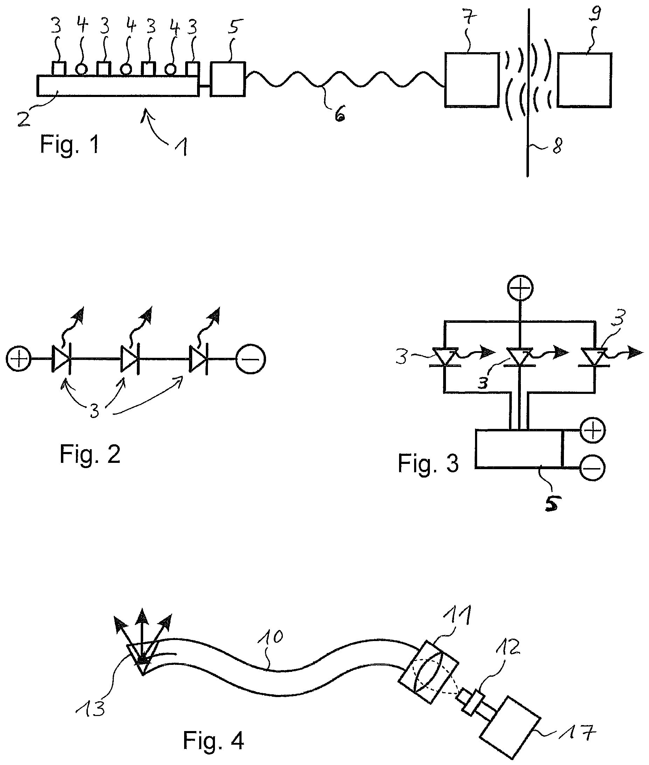

Die Figur 1 zeigt ein Beispiel eines allgemeinen Systemaufbaus der optischen Stimulationseinrichtung. Erkennbar ist eine z.B. als Stent oder Katheter ausgebildete Trägerstruktur 2, an der Lichtquellen 3 und Oberflächenelektroden 4 befestigt sind. Hierdurch wird eine Implantat-Komponente 1 bereitgestellt. Die Implantat-Komponente 1 weist eine direkt damit verbundene Headstage-Elektronik 5 auf, die eine Signalkonditionierung und Verarbeitung für ein- und ausgehende Signale vornimmt, z.B. Impedanzwandlung, Verstärkung, Digitalisierung, Ansteuerung der Lichtquellen. Hierbei können Lichtquellen 3 z.B. in Form von LEDs direkt auf der Trägerstruktur 2 angeordnet sein, oder in Form von Laserdioden in der Headstage-Elektronik 5. Deren Licht wird dann über Lichtleiter zu den in der Figur mit den Lichtquellen 3 dargestellten Positionen herausgeführt.

Die Headstage-Elektronik 5 ist über Leitungen 6 mit einer Hauptelektronik 7 des Systems verbunden. Die Hauptelektronik 7 übernimmt die Datenkommunikation mit externen Geräten 9 durch die Haut 8 und liefert Strom für den Betrieb der Lichtquellen und der Headstage-Elektronik 5. Die Hauptelektronik 7 kann ebenfalls die Interpretation der Signale und eine mögliche Rückkopplung an die Lichtquellen durchführen, sowie Daten intern speichern und auswerten. Sofern Laserdioden eingesetzt werden, können diese auch in der Hauptelektronik angeordnet sein und über Lichtleiter mit der Implantat-Komponente 1 verbunden sein.

Die Headstage-Elektronik 5 sowie die Hauptelektronik 7 bilden zusammen eine Steuerelektronik der Stimulationseinrichtung. Diese weist einen Parameterspeicher auf, in dem für jede Lichtquelle 3 ein Speicherplatz, z.B. in Form eines Bits oder Bytes, vorgesehen ist. In diesem Speicherplatz kann für jede Lichtquelle individuell eine Information gespeichert werden, ob die Lichtquelle zu Stimulationszwecken aktivierbar ist oder nicht.

Wie in Figur 2 erkennbar ist, können die Lichtquellen 3 bzw. deren Laserdioden in Serie geschaltet sein, was den Vorteil hat, dass die Verkabelung einfacher wird und weniger Leitungen erforderlich sind.

Wie in Figur 3 erkennbar ist, können die Lichtquellen 3 bzw. deren Laserdioden auch einzeln durch die Headstage-Elektronik 5 und/oder die Hauptelektronik 7 gesteuert werden. Dies hat den Vorteil, dass die Stimulation auf einen bestimmten Bereich beschränkt werden kann, indem nur einige der vorhandenen Lichtquellen aktiviert werden.

Die Figur 4 zeigt den Systemaufbau bei Verwendung von Lichtleitern. Als Lichtquelle ist ein optisches Bauteil 13 vorgesehen, das am Ende eines Lichtleiters 10 angeordnet ist. Das optische Bauteil 13 kann z.B. ein Mikrospiegel, ein Prisma oder ein ähnliches die Abstrahlrichtung änderndes optisches Bauteil sein. Das optische Bauteil 13 reflektiert das Licht in einem Winkel in das Körpergewebe, welches durch den Lichtleiter 10 und ggf. eine eingangsseitig an dem Lichtleiter 10 angeordnete Optik 11 durch einen Laseremitter 12 bereitgestellt wird. Für die Ansteuerung des Laseremitters 12 ist eine Treiberelektronik 17 vorgesehen, die z.B. Teil der Hauptelektronik 7 sein kann. Durch die Verwendung von Lichtleitern wird die optische Stimulationseinrichtung insbesondere im Hinblick auf die MRT-Tauglichkeit verbessert.

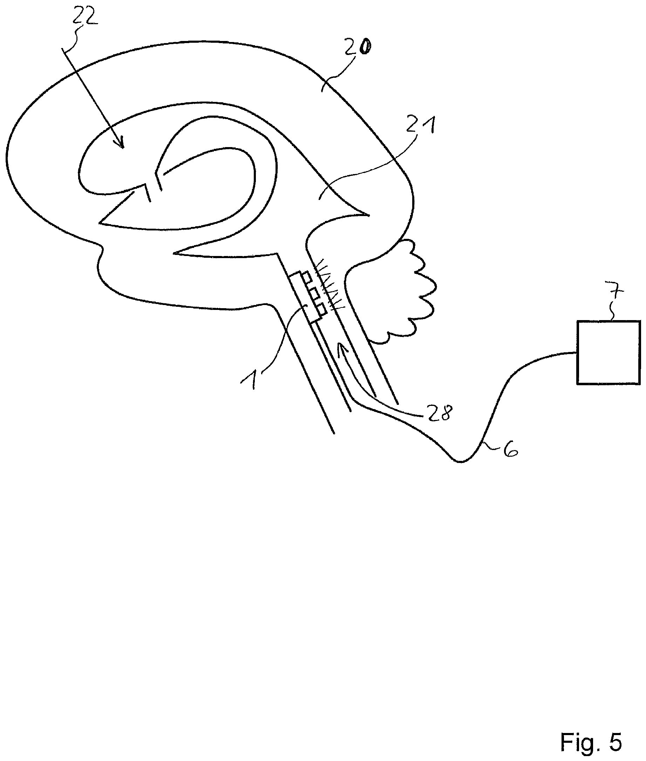

Die Figur 5 zeigt den Aufbau und die Verankerung der optischen Stimulationseinrichtung im Kopf eines Menschen. Die Implantat-Komponente 1 mit den Lichtquellen 3 und den Elektroden 4 wird in einem Ventrikelraum 21 platziert und kann von der dort vorhandenen Cerebrospinalflüssigkeit zur Kühlung der Lichtquellen umspült werden. Die Implantat-Komponente und insbesondere dessen Trägerstruktur sind hinreichend permeabel für Flüssigkeiten, um keine Stauung hervorzurufen. Die Implantat-Komponente ist über die Leitungen 6 mit der Hauptelektronik 7 verbunden. Die Hauptelektronik 7 kann unter der Haut an einer geeigneten Stelle des Körpers implantiert werden, insbesondere an einer anderen Stelle als im Kopf. Die Implantation der Implantat-Komponente 1 im Gehirn 20 kann minimalinvasiv erfolgen. Die Implantat-Komponente 1 kann z.B. über einen Weg 22 durch gesundes Gewebe an einer Stelle eingebracht werden, an der kaum Nebenwirkungen zu erwarten sind. Ebenfalls möglich ist eine Implantation über eine der natürlichen Öffnungen des Ventrikelsystems ohne Beschädigung des Gehirns 20, d.h. über den dargestellten Weg 28.

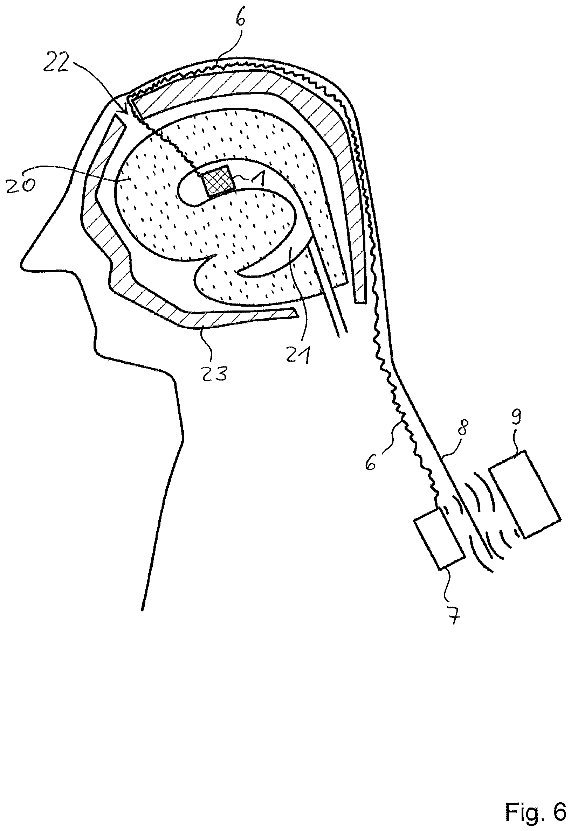

Die Figur 6 zeigt die Platzierung der Implantat-Komponente 1 im Kopf. Die Implantat-Komponente 1 befindet sich in einem Ventrikelraum 21 des Gehirns 20. Eine Verbindung für die Leitungen 6 ist durch eine Bohrung 22 im Schädel 23 oder eine natürliche Schädelöffnung nach außen gewährleistet. Die Hauptelektronik 7 befindet sich unter der Haut 8, nicht notwendigerweise unter der des Schädels 23. Die Hauptelektronik 7 kann mit externen Geräten 9 transdermal kommunizieren.

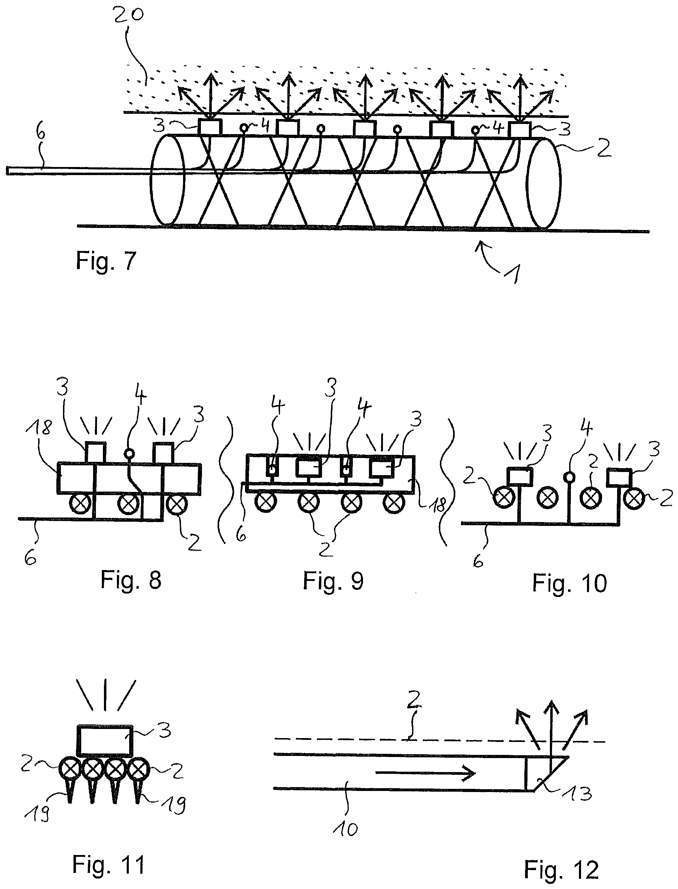

Die Figur 7 zeigt mit weiteren Details eine Ausführungsform der Implantat-Komponente 1. Eine gitterstrukturartig aufgebaute Trägerstruktur 2, die die Lichtquellen 3 und die Elektroden 4 trägt, ist im Ventrikelraum neben dem Gehirn 20 platziert. Die Trägerstruktur 2 ist hinreichend durchlässig für Cerebrospinalflüssigkeit, indem sie mit Öffnungen und/oder Kanälen ausgebildet ist. Auf und/oder in der Trägerstruktur 2 befinden sich organische oder kristalline Halbleiterlichtquellen 3 und Oberflächenelektroden 4, die durch Leitungen 6 kontaktiert werden. Durch die Pfeile ist die Lichtabstrahlung der Lichtquellen 3 in radialer Richtung bezüglich der Trägerstruktur gekennzeichnet.

Die Figur 8 bis 10 zeigen mögliche Anbringungen der Lichtquellen 3 und der Elektroden 4 an der Trägerstruktur 2. In der Figur 8 ist wiedergegeben, dass die Lichtquellen 3 und Elektroden 4 auf der Oberfläche eines flexiblen Trägermaterials 18, das zudem thermisch hinreichend leitfähig ist, aufgebracht sind, z.B. auf einem Silikonmaterial, z.B. in Form eines Silikonschlauchs, oder auf einer MEMS-Struktur. Innerhalb des Trägermaterials 18 befindet sich die Trägerstruktur 2. Die Trägerstruktur 2 kann z.B. in Form eines Stents ausgebildet sein, die eine Expansion zur Verankerung der Implantat-Komponenten im inneren Hohlraum des Körpers erlaubt. Die Leitungen 6 können im Inneren der Trägerstruktur 2 verlaufen. Alternativ kann das flexible Trägermaterial 18 auch an der Innenseite der Trägerstruktur 2 angeordnet sein.

In der Figur 9 ist erkennbar, dass die Lichtquellen 3 und die Elektroden 4 sowie die Leitungen 6 in Dünnschicht- oder Dickschichttechnik in das thermisch leitfähige flexible Trägermaterial 18 eingearbeitet sind.

Der Figur 10 zeigt, dass die Elektroden 4 und die Lichtquellen 3 direkt an der Trägerstruktur 2 befestigt sein können und dementsprechend die Formänderung (Expansion) mitmachen.

Die Figur 11 zeigt eine Möglichkeit zur zusätzlichen Kühlung der Lichtquellen über die Cerebrospinalflüssigkeit. Die Lichtquellen 3 können mit kleinen thermisch leitfähigen Kühlstrukturen (Kühlkörper) 19 ausgestattet sein, welche die entstehende Wärme an die Cerebrospinalflüssigkeit abgeben. Hierbei kann zusätzlich das Material der Trägerstruktur 2 unterstützend wirken, sofern es hinreichen wärmeleitfähig ist.

Die Trägerstruktur 2 kann vorteilhaft z.B. aus einem metallischen Material gebildet sein, ähnlich wie übliche Stents. Hierdurch kann die Wärmeableitung weiter verbessert werden.

Die Figur 12 zeigt den Einsatz von optischen Fasern (Lichtleitern 10) in Verbindung mit optischen Elementen 13 als Lichtquellen. Um eine MRT-Tauglichkeit und zusätzlich um höhere optische Leistungen zu erreichen, können Glasfasern als Lichtleiter 10 eingesetzt werden. Um das Licht in das umgebende Gewebe zu leiten, sind solche Lichtleiter 10 am Ende mit optischen Elementen 13 wie Mikrospiegeln bzw. Prismen ausgestattet, welche eine Umlenkung in die radiale Lichtabstrahlrichtung bewirken. Das Licht kann dann durch die offene Trägerstruktur 2 nach außen abgestrahlt werden, d.h. in radialer Richtung bezüglich der Trägerstruktur.

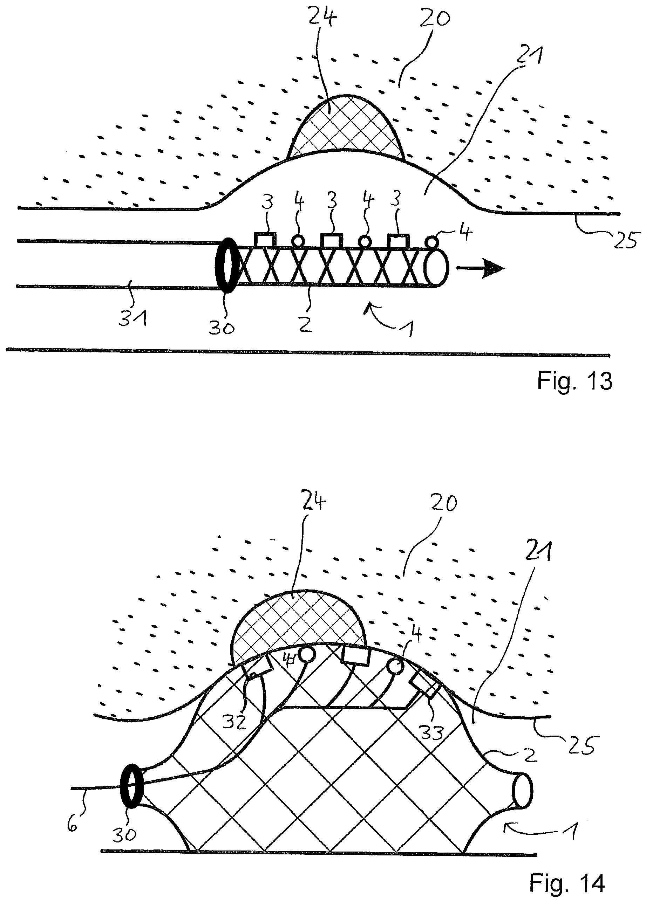

Die Figur 13 zeigt die Einbringung der Implantat-Komponente 1 mittels eines flexiblen Führungsstabs 31, z.B. eines Endoskops oder eines Katheters. Der Führungsstab 31 ist mit einem Kupplungselement 30 der Implantat-Komponente mechanisch gekoppelt. Über einen Entriegelungsmechanismus, der an dem flexiblen Führungsstab und/oder an der Implantat-Komponente angeordnet sein kann, kann diese Kopplung aufgehoben werden, um den Führungsstab 31 nach Platzierung der Implantat-Komponente 1 wieder zu entfernen. Dies erfolgt nach Expansion und dementsprechender Verankerung der Implantat-Komponente in dem inneren Hohlraum, hier im Ventrikelraum 21, wie in der Figur 14 dargestellt.

Wie aus der Figur 14 zusätzlich erkennbar ist, soll mittels der Implantat-Komponente eine Stimulation von Nervenzellen 24 erfolgen. Hierfür wird die Implantat-Komponente 1 innerhalb des Gehirns 20 mittels des Führungsstabs 31 zunächst grob an der richtigen Stelle positioniert. Anschließend wird die Trägerstruktur 2 expandiert, z.B. durch Ballonexpansion. Hierdurch werden die Lichtquellen 3 und die Elektroden 4 gegen die Wand 25 des Ventrikelraums 21 gedrückt, ohne diese zu beschädigen. Der Führungsstab 31 wird entfernt. Es verbleibt die Leitung 6 zur Verbindung der Implantat-Komponente 1 mit der Hauptelektronik 7. Es erfolgt dann eine Programmierung des Parameterspeichers der Stimulationseinrichtung derart, dass z.B. die Lichtquelle 32 als "aktivierbar" gespeichert wird und die Lichtquelle 33 als "nicht aktivierbar". Die Nervenzellen 24 können dann durch die aktivierbaren Lichtquellen 32 stimuliert werden. Die weiter entfernt liegenden, nicht aktivierbaren Lichtquellen 33 können zur Energieeinsparung ausgeschaltet bleiben. Die Identifikation der aktivierbaren Lichtquellen kann durch probeweises Aktivieren einzelner Lichtquellen und durch Messung der Reaktion der Nervenzellen 24 erfolgen. Die Messung kann über die Elektroden 4 selbst oder durch eine anderweitig gemessene Antwort durchgeführt werden, z.B. durch EEG, Verhaltensantwort, funktionelle Kernspintomographie, PET, SPECT, pharmakologische Methoden.

## Patentansprüche

1. Optische Stimulationseinrichtung zur Stimulation von Nervenzellen, wobei die Stimulationseinrichtung wenigstens eine Implantat-Komponente (1) aufweist, die zum Implantieren in einem von einer Körperflüssigkeit durchflossenen natürlichen inneren Hohlraum (21) des Körpers eines Lebewesens eingerichtet ist, mit folgenden Merkmalen:
a) die Implantat-Komponente (1) weist wenigstens eine Trägerstruktur (2) auf, die zur Fixierung in dem natürlichen inneren Hohlraum (21) in radialer Richtung expandierbar ist,
b) an der Trägerstruktur (2) ist eine Vielzahl von Lichtquellen (3, 13) befestigt, die zur Lichtabstrahlung in radialer Richtung bezüglich der Trägerstruktur (2) eingerichtet sind,
c) an der Trägerstruktur (2) sind eine Vielzahl von Elektroden (4) befestigt, die zur Erfassung elektrischer Körpersignale eingerichtet sind,
d) die Trägerstruktur (2) weist mehrere Öffnungen und/oder Kanäle auf, die nach Implantation im Körper von Körperflüssigkeit durchströmbar sind, sodass die Lichtquellen kühlbar sind, indem sie von der Körperflüssigkeit umspült werden.

2. Stimulationseinrichtung nach Anspruch 1, **dadurch gekennzeichnet, dass** die Implantat-Komponente (1) eine Steuerelektronik (5, 7) aufweist und/oder eine entfernt von der Implantat-Komponente (1) anordenbare und mit dieser über Leitungen (6) verbindbare Steuerelektronik (5, 7) aufweist, wobei die Steuerelektronik (5, 7) zur Signalkonditionierung und/oder Signalverarbeitung der über die Elektroden (4) aufgenommenen Signale und/oder zur Ansteuerung der Lichtquellen (3) eingerichtet ist.

3. Stimulationseinrichtung nach dem vorhergehenden Anspruch, **dadurch gekennzeichnet, dass** die Steuerelektronik (5, 7) einen Parameterspeicher aufweist, in dem für jede Lichtquelle (3) eine Information gespeichert ist, ob die Lichtquelle (3) zu Stimulationszwecken aktivierbar ist oder nicht.

4. Stimulationseinrichtung nach dem vorhergehenden Anspruch, **dadurch gekennzeichnet, dass** die Lichtquellen (3) in vordefinierter Lage bezüglich der Elektroden (4) an der Trägerstruktur (2) befestigt sind.

5. Stimulationseinrichtung nach einem der vorhergehenden Ansprüche, **dadurch gekennzeichnet, dass** die Trägerstruktur (2) im nichtexpandierten Zustand einen Umfang von weniger als 10 mm aufweist, insbesondere weniger als 6 mm.

6. Stimulationseinrichtung nach einem der vorhergehenden Ansprüche, **dadurch gekennzeichnet, dass** die Trägerstruktur (2) ein Material mit hoher Wärmeleitfähigkeit aufweist, insbesondere mit einer Wärmeleitfähigkeit größer als 10 W/m·K.

7. Stimulationseinrichtung nach einem der vorhergehenden Ansprüche, **dadurch gekennzeichnet, dass** eine, mehrere oder alle Lichtquellen (3) thermisch leitfähige Kühlkörper (19) aufweisen.

8. Stimulationseinrichtung nach einem der vorhergehenden Ansprüche, **dadurch gekennzeichnet**, das die Implantat-Komponente (1) ein Kupplungselement (30) aufweist, über das die Implantat-Komponente (1) mit einem zur Durchführung des Implantationsvorgangs mit der Implantat-Komponente (1) zu koppelnden flexiblen Führungsstab (31) koppelbar ist, wobei die Kopplung des Kupplungselements (30) mit dem flexiblen Führungsstab (31) durch Betätigung eines Entriegelungsmechanismus lösbar ist.

9. Stimulationseinrichtung nach einem der vorhergehenden Ansprüche, **dadurch gekennzeichnet, dass** einige oder alle Lichtquellen (3) individuell aktivierbar sind.

10. Stimulationseinrichtung nach einem der vorhergehenden Ansprüche, **dadurch gekennzeichnet, dass** die Lichtquellen (3) und/oder die Elektroden (4) auf einem flexiblen Trägermaterial (18) befestigt sind, das an der Trägerstruktur (2) befestigt ist.

11. Verfahren zur Programmierung des Parameterspeichers einer Stimulationseinrichtung nach Anspruch 3, **dadurch gekennzeichnet, dass** zunächst alle Lichtquellen (3) zur Lichtabgabe betätigt werden, die daraufhin mittels der Elektroden (4) erfassten Signale ausgewertet werden oder durch andere Nervensignal-Erfassungsmethoden erfasste Signale ausgewertet werden, hieraus zukünftig aktivierbare Lichtquellen (3) aus der Gesamtmenge der vorhandenen Lichtquellen (3) bestimmt werden und für diese Lichtquellen (3) im Parameterspeicher die Kennung "aktivierbar" gespeichert wird und für alle übrigen Lichtquellen (3) die Kennung "nicht-aktivierbar" gespeichert wird.

## Claims

1. An optical stimulation device for stimulating nerve cells, the stimulation device having at least one implant component (1) configured for implantation in a natural inner cavity (21) of the body of a living organism, through which cavity a body fluid flows, having the following features:
a) the implant component (1) has at least one support structure (2) which is expandable in the radial direction for fixation in the natural inner cavity (21),
b) attached to the support structure (2) is a multiplicity of light sources (3, 13) which are configured for radiation of light in the radial direction with respect to the support structure (2),
c) attached to the support structure (2) is a multiplicity of electrodes (4) which are configured for capturing electric body signals,
d) the support structure (2) has multiple openings and/or channels through which the body fluid can flow after implantation in the body, such that the light sources can be cooled by bathing them in the body fluid.

2. The stimulation device as claimed in claim 1, **characterized in that** the implant component (1) has a control electronics system (5, 7) and/or has a control electronics system (5, 7) arranged distantly from the implant compound (1), which is connected via lines (6) to control electronics system (5, 7), the control electronics system (5, 7) being configured for signal conditioning and/or signal processing of the signals received via the electrodes (4) and/or for activating the light sources (3).

3. The stimulation device as claimed in the preceding claim, **characterized in that** the control electronics system (5, 7) has a parameter memory in which an item of information is stored for each light source (3), regarding whether the light source (3) is activatable or not for the purposes of stimulation.

4. The stimulation device as claimed in the preceding claim, **characterized in that** the light sources (3) are attached to the support structure (2) in a predefined position with respect to the electrodes (4) .

5. The stimulation device as claimed in any of the preceding claims, **characterized in that** the support structure (2) has, in the nonexpanded state, a circumference of less than 10 mm, more particularly less than 6 mm.

6. The stimulation device as claimed in any of the preceding claims, **characterized in that** the support structure (2) comprises a material having a high thermal conductivity, more particularly having a thermal conductivity greater than 10 W/m·K.

7. The stimulation device as claimed in any of the preceding claims, **characterized in that** one, multiple or all light sources (3) have thermally conductive heat sinks (19).

8. The stimulation device as claimed in any of the preceding claims, **characterized in that** the implant component (1) has a coupling element (30) via which the implant component (1) is coupleable to a flexible guide rod (31) to be coupled to the implant component (1) for carrying out the implantation process, the coupling of the coupling element (30) to the flexible guide rod (31) being disengageable by actuation of a release mechanism.

9. The stimulation device as claimed in any of the preceding claims, **characterized in that** some or all light sources (3) are individually activatable.

10. The stimulation device as claimed in any of the preceding claims, **characterized in that** the light sources (3) and/or the electrodes (4) are attached on a flexible support material (18) which is attached to the support structure (2).

11. A method for programming the parameter memory of a stimulation device as claimed in claim 3, **characterized in that** all light sources (3) for light emission are actuated first of all, the signals subsequently captured by means of the electrodes (4) are evaluated or signals captured by other nerve-signal capture methods are evaluated, light sources (3) from the total of the light sources (3) present that will be activatable in future are determined therefrom, and the identification "activatable" is stored in the parameter memory for these light sources (3) and the identification "nonactivatable" is stored for all other light sources (3)

## Revendications

1. Dispositif de stimulation optique destiné à stimuler des cellules nerveuses, le dispositif de stimulation comportant au moins un composant sous forme d'implant (1) qui est aménagé pour être implanté dans une cavité interne naturelle (21) du corps d'un être vivant irriguée par un fluide corporel, présentant les caractéristiques suivantes :
a) le composant sous forme d'implant (1) comporte au moins une structure porteuse (2), qui pour la fixation dans la cavité interne naturelle (21) est expansible en direction radiale,
b) sur la structure porteuse (2) est fixée une pluralité de sources lumineuses (3, 13) qui sont aménagées pour émettre de la lumière dans la direction radiale, par rapport à la structure porteuse (2),
c) sur la structure porteuse (2) est fixée une pluralité d'électrodes (4) qui sont aménagées pour détecter des signaux corporels électriques,
d) la structure porteuse (2) comporte plusieurs orifices et/ou conduits, qui après l'implantation dans le corps peuvent être irrigués par un fluide corporel, de telle sorte que les sources lumineuses puissent être refroidies en étant baignées dans un flot de fluide corporel.

2. Dispositif de stimulation selon la revendication 1, **caractérisé en ce que** le composant sous forme d'implant (1) comporte un système de commande électronique (5, 7) et/ou comporte un système de commande électronique (5, 7) pouvant être placé loin du composant sous forme d'implant (1) et connecté avec ce dernier par l'intermédiaire de lignes (6), le système de commande électronique (5, 7) étant aménagé pour le conditionnement de signaux et/ou pour le traitement de signaux des signaux perçus via les électrodes (4) et/ou pour l'amorçage des sources lumineuses (3).

3. Dispositif de stimulation selon la revendication précédente, **caractérisé en ce que** le système de commande électronique (5, 7) comporte une mémoire de paramètres, dans laquelle pour chaque source lumineuse (3) est mémorisée une information indiquant si la source lumineuse (3) est activable ou non à des fins de stimulations.

4. Dispositif de stimulation selon la revendication précédente, **caractérisé en ce que** les sources lumineuses (3) sont fixées sur la structure porteuse (2), dans une position prédéfinie par rapport aux électrodes (4).

5. Dispositif de stimulation selon l'une quelconque des revendications précédentes, **caractérisé en ce qu'**à l'état non expansé, la structure porteuse (2) présente une périphérie inférieure à 10 mm, notamment inférieure à 6 mm.

6. Dispositif de stimulation selon l'une quelconque des revendications précédentes, **caractérisé en ce que** la structure porteuse (2) comporte une matière à haute conductivité thermique, notamment d'une conductivité thermique supérieure à 10 W/m·K.

7. Dispositif de stimulation selon l'une quelconque des revendications précédentes, **caractérisé en ce qu'**une, plusieurs ou toutes les sources lumineuses (3) comportent des corps de refroidissement (19) aptes à la conductibilité thermique.

8. Dispositif de stimulation selon l'une quelconque des revendications précédentes, **caractérisé en ce que** le composant sous forme d'implant (1) comporte un élément de couplage (30) par l'intermédiaire duquel le composant sous forme d'implant (1) peut se coupler sur une tige de guidage (31) flexible qui doit être couplée sur le composant sous forme d'implant (1) pour la réalisation du processus d'implantation, le couplage de l'élément de couplage (30) avec la tige de guidage (31) flexible étant désolidarisable par actionnement d'un mécanisme de déverrouillage.

9. Dispositif de stimulation selon l'une quelconque des revendications précédentes, **caractérisé en ce que** certaines ou toutes les sources lumineuses (3) sont activables individuellement.

10. Dispositif de stimulation selon l'une quelconque des revendications précédentes, **caractérisé en ce que** les sources lumineuses (3) et/ou les électrodes (4) sont fixées sur une matière porteuse (18) flexible qui est fixée sur la structure porteuse (2).

11. Procédé, destiné à programmer la mémoire de paramètres d'un dispositif de stimulation selon la revendication 3, **caractérisé en ce que** d'abord toutes les sources lumineuses (3) sont actionnées pour l'émission de lumière, les signaux qui par la suite sont détectés au moyen des électrodes (4) ou des signaux détectés via d'autres méthodes de détection de signaux nerveux sont évalués, suite à quoi, des sources lumineuses (3) activables dans le futur sont déterminées parmi la quantité totale des sources lumineuses (3) présentes et pour lesdites sources lumineuses (3), dans la mémoire de paramètres est mémorisée l'identification « activable » et pour toutes les sources lumineuses (3) restantes est mémorisée l'identification « non activable ».
